# EUROPEAN PATENT APPLICATION

(11) **EP 0 639 568 A1**
(43) Date of publication of application: **22.02.1995**
(21) Application number: 93610047.8
(22) Date of filing: 19.08.1993
(51) Int. Cl.: C07D 211/22, A61K 31/445, A61K 31/495, A61K 31/535, C07D 211/26, C07D 401/06

(54) **Piperidine compounds, their preparation and use in the treatment of neurodegenerative disorders**

(71) Applicant: NOVO NORDISK A/S, DK-2880 Bagsvaerd (DK)
(72) Inventor: Lundbeck, Jane Marie, DK-2600 Glostrup (DK); Kanstrup, Anders, DK-2830 Virum (DK); Jakobsen, Palle, DK-3500 Vaerlose (DK)

(57) **Abstract**

The novel compounds are useful in the treatment of anoxia, traumatic injury, ischemia, migraine, epilepsy, Parkinson's disease, Alzheimer's disease and other neurodegenerative diseases, are of formula I 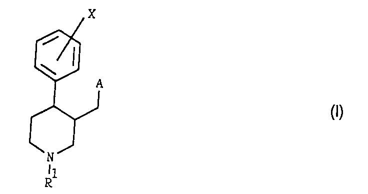
wherein
A is 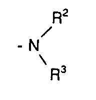
or - O - R⁴
wherein R² and R³ independently are hydrogen, straight or branched C₁#SB-#₆-alkyl optionally substituted with an amino group which amino group is optionally mono of disubstituted with straight or branched C₁#SB-#₆-alkyl, straight or branched C₂#SB-#₆-alkenyl or straight or branched C₂#SB-#₆-alkynyl; or R² and R³ together form a 5 or 6 membered heterocyclic ring containing one to four N, O or S atom(s) or a combination thereof which heterocyclic ring is optionally substituted at a carbon or nitrogen atom with straight or branched C₁#SB-#₆-alkyl, straight or branched C₂#SB-#₆-alkenyl, straight or branched C₂#SB-#₆-alkynyl or phenyl which phenyl group is optionally substituted with halogen, C₁#SB-#₄-alkyl, C₁#SB-#₄-alkoxy or trifluoromethyl, or one or two carbon atom(s) in the heterocyclic ring together with one or two oxygen atom(s) form one or two carbonyl group(s), or which heterocyclic ring is optionally fused with a benzo group; and
R⁴ is straight or branched C₁#SB-#₆-alkyl optionally substituted with an amino group which amino group is optionally mono or disubstituted with straight or branched C₁#SB-#₆-alkyl, straight or branched C₂#SB-#₆-alkenyl or C₂#SB-#₆-alkynyl; and R¹ is hydrogen, straight or branched C₁#SB-#₆-alkyl optionally substituted with halogen, C₁#SB-#₆-alkoxy, phenyl or phenoxy, straight or branched C₂#SB-#₆-alkenyl or straight or branched C₂#SB-#₆-alkynyl; and
X is hydrogen, halogen, cyano, C₁#SB-#₄-alkyl, C₁#SB-#₄-alkoxy, hydroxy, nitro, trifluoromethyl or an amino group optionally mono or disubstituted with straight or branched C₁#SB-#₆-alkyl, or a pharmaceutically acceptable salt thereof.

## Description

The present invention relates to therapeutically active piperidine compounds, a method of preparing the same and to pharmaceutical compositions comprising the compounds. The novel compounds are useful in the treatment of anoxia, traumatic injury, ischemia, migraine, epilepsy, Parkinson's disease, Alzheimer's disease and other neurodegenerative diseases.

It is well known that accumulation of calcium in the brain cells (calcium overload) is seen after periods of uncontrolled hyperactivity in the brain, such as after convulsions, migraine, anoxia and ischemia. As the concentration of calcium in the cells is of vital importance for the regulation of cell function, and uncontrolled high concentration of the cell calcium will lead to, or indirectly cause the symptoms and possibly also the degenerative changes combined with the above diseases.

Therefore, calcium overload blockers selective for brain cells will be useful in the treatment of anoxia, traumatic injury, ischemia, migraine, epilepsy, Parkinson's disease, Alzheimer's disease and other neurodegenerative diseases.

Well known calcium antagonists such as nifedipine, verapamil and diltiazem have activity against peripheral calcium uptake, e.g. in blood vessels and the heart, however, they have shown only very low activity against calcium overload in brain cells.

Accordingly it is an object of the invention to provide novel compounds having activity against calcium overload in brain cells.

The novel compounds of the invention are of formula I

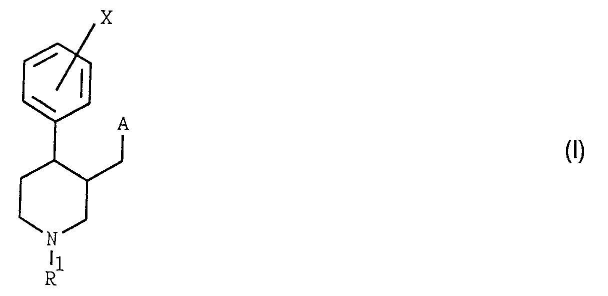

wherein
A is 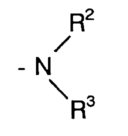
or - O - R⁴
wherein R² and R³ independently are hydrogen, straight or branched C₁₋₆-alkyl optionally substituted with an amino group which amino group is optionally mono or disubstituted with straight or branched C₁₋₆-alkyl, straight or branched C₂₋₆-alkenyl or straight or branched C₂₋₆-alkynyl; or
R² and R³ together form a 5 or 6 membered heterocyclic ring containing one to four N, O or S atom(s) or a combination thereof which heterocyclic ring is optionally substituted at a carbon or nitrogen atom with straight or branched C₁₋₆-alkyl, straight or branched C₂₋₆-alkenyl, straight or branched C₂₋₆-alkynyl or phenyl which phenyl group is optionally substituted with halogen, C₁₋₄-alkyl, C₁₋₄-alkoxy or trifluoromethyl, or one or two carbon atom(s) in the heterocyclic ring together with one or two oxygen atom(s) form one or two carbonyl group(s), or which heterocyclic ring is optionally fused with a benzo group; and
R⁴ is straight or branched C₁₋₆-alkyl optionally substituted with an amino group which amino group is optionally mono or disubstituted with straight or branched C₁₋₆-alkyl, straight or branched C₂₋₆-alkenyl or C₂₋₆-alkynyl; and R¹ is hydrogen, straight or branched C₁₋₆-alkyl optionally substituted with halogen, C₁₋₆-alkoxy, phenyl or phenoxy, straight or branched C₂₋₆-alkenyl or straight or branched C₂₋₆-alkynyl; and
X is hydrogen, halogen, cyano, C₁₋₄-alkyl, C₁₋₄-alkoxy, hydroxy, nitro, trifluoromethyl or an amino group optionally mono or disubstituted with straight or branched C₁₋₆-alkyl, or a pharmaceutically acceptable salt thereof.

Examples of such salts include inorganic and organic acid addition salts such as hydrochloride, hydrobromide, sulphate, phosphate, acetate, fumarate, maleate, citrate, lactate, tartrate, oxalate, or similar pharmaceutically acceptable inorganic or organic acid addition salts.

The invention also relates to a method of preparing the above mentioned compounds. These methods comprise
a) reacting a compound of formula II 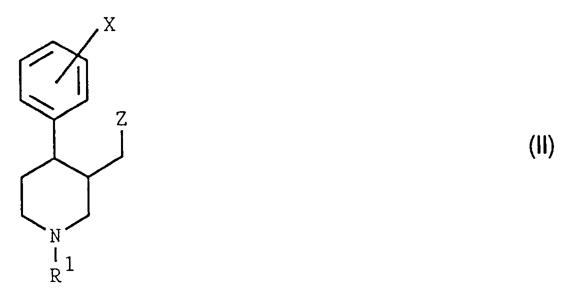 wherein X and R¹ have the meanings defined above provided that R¹ is not hydrogen and Z is a leaving group such as e.g. halogen or a sulfonate, with a compound of formula III 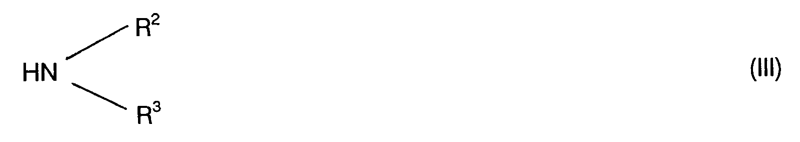 wherein R² and R³ have the meanings defined above; or
b) reacting a compound of formula II 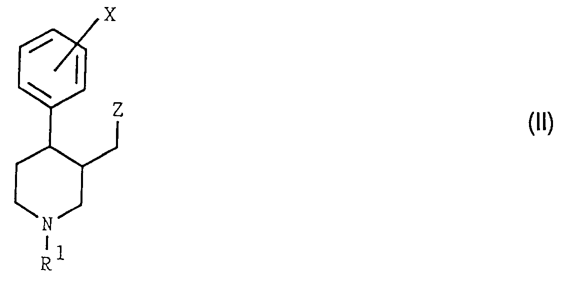 wherein X and R¹ have the meanings defined above provided that R¹ is not hydrogen and Z is a leaving group such as e.g. halogen or a sulfonate, with a compound of formula IV

   R⁴ - OH (IV)

   wherein R⁴ has the meaning defined above; or
c) demethylating a compound of formula I wherein R¹ is methyl by means of ROCOCl wherein R preferably are -CHClCH₃, -CH=CH₂ and -CH₂CCl₃ to form a compound of formula I wherein R¹ is hydrogen; or
d) reacting a compound of formula V 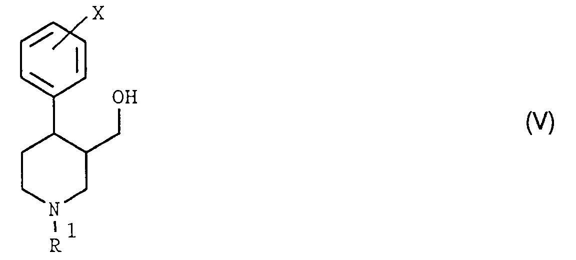 wherein X and R¹ have the meanings defined above with a compound of formula III 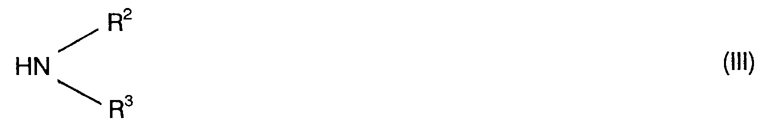 wherein R² and R³ have the meanings defined above by means of Ph₃P and (EtOCON)₂; or
e) reacting a compound of formula V 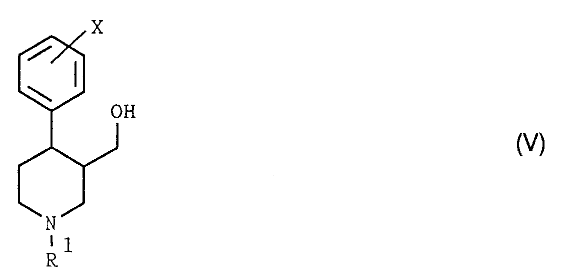 wherein X and R¹ have the meanings defined above with a compound of formula IV

   R⁴ - OH (IV)

   wherein R⁴ has the meaning defined above by means of Ph₃P and (EtOCON)₂; or
f) reacting a compound of formula VI 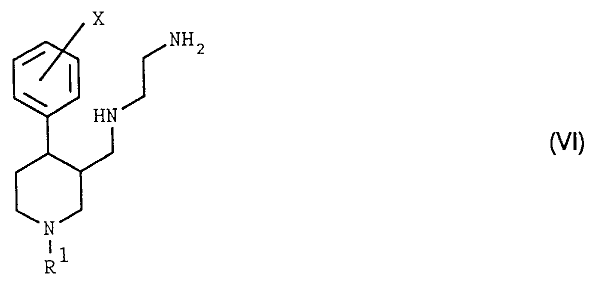 wherein X and R¹ have the meanings defined above provided that R¹ is not hydrogen with a compound of formula VII

   R² - Y (VII)

   wherein R² is straight or branched C₁₋₆-alkyl and Y is a leaving group such as e.g. halogen or a sulfonate; or
g) reacting a compound of formula VI 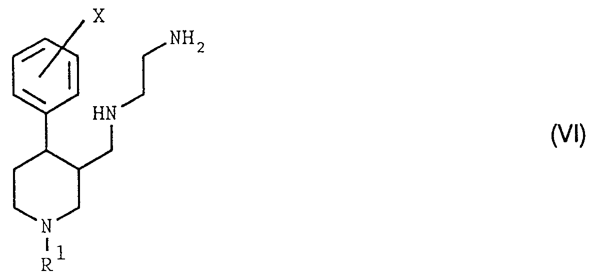 wherein X and R¹ have the meanings defined above provided that R¹ is not hydrogen with a compound of formula VIII

   Y-CH₂CH₂-Y (VIII)

   wherein Y is a leaving group such as e.g. halogen or a sulfonate.

The pharmacological properties of the compounds of the invention can be illustrated by determining their capaility to inhibit calcium uptake into brain synaptosomes.

### PRINCIPLE

Depolarization of neuronal membranes leads to an opening of socalled "voltage operated calcium channels" (VOC) in the membranes which allows a massive influx of calcium from the extracellular space. A crude synaptosomal preparation (socalled P₂ fraction) contains small vesicles surrounded by neuronal membrane and it is possible in such a preparation to study a depolarization-induced opening of VOC. In the present model ⁴⁵Ca influx is induced in the synaptosomes by depolarization with elevated potassium concentrations, and the effect of test substances on this stimulated uptake is studied (Nachshen, D.A. and Blaustein, M.P., Mol. Pharmcol., 16, 579 (1979)).

### ASSAY

A male Wistar rat is decapitated and the cerebral cortex removed and homogenized in 10 ml of ice-cold 0.32 M sucrose using a glass homogenizer with a teflon pestle. All subsequent steps for isolation of synaptosomes are done at 0-4°C. The homogenate is centrifuged at 1000 x g for 10 min. and the resulting supernatant is re-centrifuged at 18000 x g for 20 min. This pellet (P₂) is resuspended in 0.32 M sucrose (5 ml per g of original tissue) with a teflon pestle.

Aliquots (0.050 ml) of this crude synaptosomal suspension are added to glass tubes containing 0.625 ml of NaCl buffer (136 mM NaCl, 4 mM KCl, 0.35 mM CaCl₂, 1.2 mM MgCl₂, 20 mM Tris HCl, 12 mM glucose, pH 7.4) and 0.025 ml of various drug solutions in 48% ethanol. The tubes are preincubated for 30 min. on ice and then for 6 min. at 37°C in a water bath.

The uptake is immediately initiated by adding 0.4 ml of ⁴⁵CaCl₂ (specific activity = 29-39 Ci/g; 0.5 µCi/assay), in 145 mM NaCl for non-depolarized samples and in 145 mM KCl for depolarized samples. The incubation is continued for 15 s.

The uptake is terminated by rapid filtration through GF-C glass fiber filters which are washed three times with 5 ml of a cold solution containing 145 mM KCl, 7 mM EGTA and 20 mM Tris HCl, pH 7.4. The amount of radioactivity on the filter disc is determined by liquid scintillation spectrometry.

### TEST PROCEDURE

Test substances are dissolved in 10 ml of 48% ethanol at a concentration of 0.44 mg/ml. Dilution is made in 48% ethanol to give final concentrations of 0.1, 0.3, 1,3 and 10 µg/ml. Experiments are performed in triplicate. Controls for depolarized and nondepolarized samples are included in the assay and test substances are only tested in depolarized samples. 25-75% inhibition of stimulated uptake must be obtained before calculating the IC₅₀ value.

### RESULTS

The test value will be given as MEC (minimal effective concentration (µg/ml)) of test substance which inhibits stimulated uptake of ⁴⁵Ca significant different (P< 0.05, Student's t-test) from control.

Test results obtained by testing some compounds of the present invention will appear from the following Table 1.

**Table 1**

| Compound No. | MEC (µg/ml) |
|---|---|
| 1 | >1 |
| 2 | 1 |
| 4 | >1 |
| 6 | 0.3 |
| 7 | 3 |
| 8 | >3 |
| 9 | >3 |
| 11 | 1 |
| 13 | >1 |
| 14 | 1 |

The compounds of the invention, together with a conventional adjuvant, carrier, or diluent, and if desired in the form of a pharmaceutically acceptable acid addition salt thereof, may be placed into the form of pharmaceutical compositions and unit dosages thereof, and in such form may be employed as solids, such as tablets or filled capsules, or liquids, such as solutions, suspensions, emulsions, elixirs, or capsules filled with the same, all for oral use, in the form of suppositories for rectal administration; or in the form of sterile injectable solutions for parenteral use (including subcutaneous administration and infusion). Such pharmaceutical compositions and unit dosage forms thereof may comprise conventional ingredients in conventional proportions, with or without additional active compounds or principles, and such unit dosage forms may contain any suitable effective calcium overload blocking amount of the active ingredient commensurate with the intended daily dosage range to be employed. Tablets containing ten (10) milligrams of the active ingredient or, more broadly, one (1) to hundred (100) milligrams, per tablet, are accordingly suitable representative unit dosage forms.

The compounds of this invention can thus be used for the formulation of pharmaceutical preparations, e.g. for oral and parenteral administration to mammals including humans, in accordance with conventional methods of galenic pharmacy.

Conventional excipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Ampoules are convenient unit dosage forms.

Tablets, dragees, or capsules having talc and/or a carbohydrate carrier or binder or the like, the carrier preferably being lactose and/or corn starch and/or potato starch, are particularly suitable for oral application. A syrup, elixir or the like can be used in cases where a sweetened vehicle can be employed.

Generally, the compounds of this invention are dispensed in unit form comprising 0.05-100 mg in a pharmaceutically acceptable carrier per unit dosage.

The dosage of the compounds according to this invention is 0.1-300 mg/day, preferably 10-100 mg/day, when administered to patients, e.g. humans, as a drug.

A typical tablet which may be prepared by conventional tabletting techniques contains:

| | |
|---|---|
| Active compound | 5.0 mg |
| Lactosum | 67.8 mg Ph.Eur. |
| Avicel® | 31.4 mg |
| Amberlite® IRP 88 | 1.0 mg |
| Magnesii stearas | 0.25 mg Ph.Eur. |

Due to the high calcium overload blocking activity, the compounds of the invention are extremely useful in the treatment of symptoms related to an accumulation of calcium in brain cells of mammals, when administered in an amount effective for calcium overload blocking activity, including both activity against anoxia, traumatic injury, ischemia, migraine, epilepsy, Parkinson's disease, Alzheimer's disease and other neurodegenerative diseases. The compounds of the invention may accordingly be administered to a subject, e.g., a living animal body, including a human, in need of a calcium overload blocker, and if desired in the form of a pharmaceutically acceptable acid addition salt thereof (such as the hydrobromide, hydrochloride, or sulfate, in any event prepared in the usual or conventional manner, e.g., evaporation to dryness of the free base in solution together with the acid), ordinarily concurrently, simultaneously, or together with a pharmaceutically acceptable carrier or diluent, especially and preferably in the form of a pharmaceutical composition thereof, whether by oral, rectal, or parenteral (including subcutaneous) route, in an effective calcium overload blocking amount, and in any event an amount which is effective for the treatment of anoxia, traumatic injury, ischemia, migraine, epilepsy, Parkinson's disease, Alzheimer's disease and other neurodegenerative diseases due to their calcium overload blocking activity. Suitable dosage ranges are 1-200 milligrams daily, 10-100 milligrams daily, and especially 30-70 milligrams daily, depending as usual upon the exact mode of administration, form in which administered, the indication towards which the administration is directed, the subject involved and the body weight of the subject involved, and the preference and experience of the physician or veterinarian in charge.

The invention will now be described in further detail with reference to the following examples:

### EXAMPLE 1

### (+-)-trans-3-((2-Aminoethyl)aminomethyl)-1-methyl-4-phenylpiperidine, HCl (1)

7.0 g (0.02 mol) (+-)-cis-3-benzenesulphonyloxymethyl-1-methyl-4-phenylpiperidine (2) was dissolved in 50 ml 1,2-diaminoethane. The reaction mixture was refluxed for 7.5 h. The reaction mixture was evaporated in vacuo. The resulting oil was dissolved in 4 N NaOH and extracted with dichloromethane (3 x 200 ml), then dried over MgSO₄, evaporation in vacuo gave 4.65 g yellow oil (92%). Some oil was acidified by conc. HCl, the resulting glass was very hygroscopic. M.p. 257-270°C.

¹H-NMR (CDCl₃,δ): 7.2 (s,5H), 2.45 (s,3H), 1.1-2.3 (m,10H), 2.5-3.2 (m,7H).

### EXAMPLE 2

### (+-)-trans-3-(N-Butyl-N-(2-dibutylamino)ethyl)aminomethyl-1-methyl-4-phenylpiperidine, HCl (3)

0.5 g (2.0 mmol) (free amine) (1) was dissolved in abs. ethanol. 1.45 g (10.58 mmol) n-bromobutane and 2 g K₂CO₃ were added together with one crystal of KI. The reaction mixture was stirred for 15 h at room temperature. Silica gel TLC (CH₂Cl₂/MeOH; 9:1) showed no reaction. Then the reaction mixture was refluxed for 72 h. The reaction mixture was filtrated. K₂CO₃ and KI was washed with ether. The ethanol-ether phase was evaporated in vacuo. The resulting oil was dissolved in 4 N NaOH, extracted with ether (3 x 50 ml), and dried over MgSO₄. Evaporation in vacuo gave 0.510 g oil, which was purified on silica gel (eluent: CH₂Cl₂/ CH₃OH; 9:1). The resulting oil (0.03 g) was acidified with conc. HCl, giving 38.4 mg (4%) white crystals of (3). M.p. 201.2-202.2°C. MS: 416 (M+1).

### EXAMPLE 3

### (+-)-trans-3-((2-Aminoethyl)aminomethyl)-4-(4-fluorophenyl)-1-pentylpiperidine, HCl (4)

5 g (0.012 mol) of (+-)-trans-3-benzenesulphonyloxymethyl-4-(4-fluorophenyl)-1-pentylpiperidine (5) was dissolved in 50 ml 1,2-diaminoethane. The reaction mixture was heated at 60°C for 22 h. H₂O was added and the mixture was evaporated in vacuo to give 7.63 g brown oil which was dissolved in 4 N NaOH and extracted with CH₂Cl₂ (3 x 200 ml). The dichloromethane phase was dried with MgSO₄, evaporated and acidified with conc. HCl. Recrystallization from CH₃OH/ether gave 2.2 g white crystals (46%). M.p. > 289°C.

### EXAMPLE 4

### (+-)-trans-3-(N-Butyl-N-(2-dibutylamino)ethyl)-aminomethyl-4-(4-fluorophenyl)-1-pentylpiperidine, HCl (6)

The compound was prepared in the same manner as described for (3). Yield 0.6 g (47%) hydrochloride as a glass.

¹H-NMR (CDCl₃,δ): 6.8-7.35 (m,4H), 8.7 (s,2H), 0.95 (t,12H), 1.2-2.0 (m,20H), 2.1-2.5 p(m,8H), 2.5-3.7 (m,12H).

### EXAMPLE 5

### (+-)-trans-4-(4-Fluorophenyl)-3-(N-propyl-N-(2-dipropylamino)ethyl)-aminomethyl-1-pentylpiperidine, HCl (7)

The compound was prepared in the same manner as described for (3). Yield 120 mg (23%) as a hard glass.

¹H-NMR (CDCl₃,δ): 6.7-7.3 (m,4H), 0.7-1.1 (broad t,12H), 1.1-1.7 (m,17H), 1.7-3.3 (m,17H).

### EXAMPLE 6

### (-)-trans-3-((3-Aminopropyl)aminomethyl)-4-(4-fluorophenyl)-1-pentylpiperidine, HCl (8)

The compound was prepared in the same manner as described for (1). Yield 1.86 g (76%) as a hard glass.

¹H-NMR (CDCl₃,δ): 6.9-7.4 (m,4H), 1.1 p(t,3H), 1.3-1.9 (m,10H), 1.9-2.3 (m,6H), 2.35-3.0 (m,7H), 3.0-3.4 (m,4H).

### EXAMPLE 7

### (-)-trans-1-Butyl-4-(4-fluorophenyl)-3-(morpholinomethyl)piperidine, HCl (9)

4.2 g (0.011 mol) of (+)-cis-3-benzenesulphonyloxymethyl-1-butyl-4-(4-fluorophenyl)piperidine (10) was dissolved in 200 ml toluene and 4.7 g (0.054 mol) 5 eq. morpholine was added. The reaction mixture was heated at 60-70°C for 120 h. 200 ml 4 N NaOH was added to wash the toluene phase. The water phase was washed with ether (3 x 50 ml). Toluene/ether fractions were dried over MgSO₄. Evaporation in vacuo gave 3.66 g yellow oil. Purification on silica gel (eluent: CH₂Cl₂/MeOH; 9:1) gave after acidification with conc. HCl 1.3 g (29%) as a hard glass.

¹H-NMR (CDCl₃,δ): 6.6-7.15 (m,4H), 0.8 (t,3H), 1.0-2.5 (m,17H), 2.5-3.6 (m,7H).

### EXAMPLE 8

### (+-)-trans-1-Butyl-3-(4-phenylpiperidinomethyl)-4-phenylpiperidine, HCl (11)

2.0 g (0.005 mol) (+-)-cis-3-benzenesulphonyloxymethyl-1-butyl-4-phenylpiperidine (12) was dissolved in 100 ml toluene; 1 g (0.0065 mol) 4-phenylpiperidine and 1 ml pyridine were added. The reaction mixture was heated for 24 h at 70°C. 100 ml 4 N NaOH was added. The water phase was extracted with (3 x 50 ml) ether. The toluene/ether phases were dried over MgSO₄, evaporation in vacuo gave 2.31 g yellow oil. Purification on silica gel (eluent: CH₂Cl₂/MeOH; 9:1) gave 1.3 g oil. Acidification with conc. HCl gave 1.4 g (61%) as a hard glass.

¹H-NMR (CDCl₃,δ): 7.15 (s,5H), 7.7 (s,5H), 1.0 (t,3H), 1.2-1.9 (m,10H), 2.0-2.3 (m,9H), 2.3-3.7 (m,6H).

### EXAMPLE 9

### (+-)-trans-1-Methyl-4-phenyl-3-(1-piperazinyl)methyl-piperidine, HCl (13)

2 g (0.008 mol) (1) was dissolved in 100 ml CH₂Cl₂; 1.7 g (0.009 mol) 1,2-dibromoethane and 2 g K₂CO₃ were added. The reaction mixture was heated for 5 h at 40°C. The mixture was stirred for 18 h at room temperature. 100 ml 4 N NaOH was added. The water phase was extracted with CH₂Cl₂ (3 x 50 ml), CH₂Cl₂ phases were dried over MgSO₄ and activated charcoal, evaporation in vacuo gave 1.85 g orange crystals (84%). 0.4 g was acidified by conc. HCl. Yield 0.3 g light orange crystals (59%). M.p. 188-190°C.

### EXAMPLE 10

### (+-)-trans-4-(4-Fluorophenyl)-3-(4-phenyl-1-piperazinylmethyl)-1-pentylpiperidine, HCl (14)

2.5 g (0.006 mol) (+-)-trans-3-benzenesulphonyloxymethyl-4-(4-fluorophenyl)-1-pentylpiperidine (5) was dissolved in 100 ml toluene; 1.46 g (0.009 mol) 1.5 eq. N-phenyl-piperazine and 1 ml pyridine were added. The reaction mixture was stirred 24 h at 90°C. 100 ml 4 N NaOH was added, and the water phase was extracted with ether (3 x 50 ml). Toluene/ether phases were dried over MgSO₄. Evaporation in vacuo gave 3.3 g semicrystalline brown oil. Purification on silica gel (eluent: CH₂Cl₂/MeOH; 9:1) gave 1.87 g oil. Acidification with conc. HCl gave 1.4 g (50%) light yellow crystals. M.p. 192.9-193.6°C.

### EXAMPLE 11

### (-)-trans-4-(4-Fluorophenyl)-3-(1,2,3,4-tetrahydro-2-isoquinolinylmethyl)-1-pentylpiperidine, 2 HCl (15)

1 g (0.0024 mol) (+)-cis-3-benzenesulphonyloxymethyl-4-(4-fluorophenyl)-1-pentylpiperidine (16) was dissolved in 25 ml 1,2,3,4-tetrahydroisoquinoline. The reaction mixture was heated for 72 h at 80°C. Evaporation in vacuo gave a semi-crystalline oil. The oil was washed with 25 ml 4 N NaOH and extraction with ether (3 x 50 ml) gave after drying with MgSO₄ 1.2 g brown oil. Purification on silica gel (eluent: ethyl acetate) gave after acidification with conc. HCl 90 mg of (15). M.p. 197-198°C.

### EXAMPLE 12

### (-)-trans-4-(4-Fluorophenyl)-3-(1,2,3,4-tetrahydro-1-quinolinylmethyl)-1-pentylpiperidine, 2 HCl (17)

1 g (0.0024 mol) of (16) was dissolved in 25 ml 1,2,3,4-tetrahydroquinoline. The reaction mixture was heated under N₂ for 20 h at 80°C. The mixture was added 25 ml 4 N NaOH and the water phase was extracted with ether (3 x 50 ml), evaporation in vacuo gave 1.3 g brown oil. Purification on silica gel (eluent: CH₂Cl₂/CH₃OH; 9:1) and two times with (eluent: ethyl acetate) gave 130 mg, acidification with conc. HCl gave 40 mg (3.5%) of (15). M.p. 57-58°C.

### EXAMPLE 13

### (-)-trans-3-(2-(Dimethylamino)ethoxymethyl)-4-(4-fluorophenyl)-1-pentylpiperidine, HCl (18)

1 g (0.0024 mol) of (16) was dissolved in 25 ml dimethylaminoethanol and 120 mg NaH was added. The reaction mixture was heated under N₂ for 24 h at 80°C. Then washed with 30 ml 4 N NaOH. The water phase was extracted with ether (3 x 50 ml). The ether phase was dried over MgSO₄. Evaporation in vacuo gave 0.7 g yellow oil. Purification on silica gel 3 times (eluent: CH₂Cl₂/CH₃OH; 8:2) gave 350 mg oil. Acidification with conc. HCl gave 270 mg (26.6%) white crystals. M.p. 235.7-236.4°C.

### EXAMPLE 14

### (+-)-trans-4-(4-Fluorophenyl)-1-methyl-3-phthalimidomethyl-piperidine, HCl (19)

3 g (13.5 mmol) of (+-)-trans-4-(4-fluorophenyl)-3-hydroxymethyl-1-methylpiperidine, 1.98 g (13.5 mmol) phthalimide and 3.52 g (13.5 mmol) triphenylphosphine were dissolved in 30 ml dry tetrahydrofuran (THF), and a solution of 2.34 g (13.5 mmol) of diethylazodicarboxylate (DEAD) in 10 ml dry THF was slowly added. After 3 days the solution was evaporated, and the residue was triturated with ether and filtrated. The filtrate was evaporated and purified twice by column chromatography on silica gel, first in dichloromethane:methanol (9:1), then in pentane:triethylamine (1:1). The eluent was evaporated, the product triturated with toluene and filtrated, redissolved in acetone and precipitated by the addition of conc. HCl to give 3.3 g of (19), (63%). M.p. 267-270°C.

### EXAMPLE 15

### (+-)-trans-4-(4-Fluorophenyl)-1-methyl-3-succinimidomethyl-piperidine, HCl (20)

3 g (13.5 mmol) of (+-)-trans-4-(4-fluorophenyl)-3-hydroxymethyl-1-methylpiperidine, 1,34 g (13.5 mmol) succinimide and 3.52 g (13.5 mmol) triphenylphosphine were dissolved in 30 ml dry tetrahydrofuran (THF), and a solution of 2.34 g (13.5 mmol) of diethylazodicarboxylate (DEAD) in 10 ml dry THF was slowly added. After 3 days the solution was evaporated, and the residue was triturated with ether and filtrated. The filtrate was evaporated and purified by column chromatography on silica gel in dichloromethane:methanol (9:1). The eluent was evaporated, and the product dissolved in acetone and precipitated by the addition of conc. HCl to give 635 mg of (20), (14%). M.p. 243-245°C.

### EXAMPLE 16

### (+-)-trans-1-Butyl-4-(4-dimethylaminophenyl)-3-phthalimidomethyl-piperidine, 2 HCl (21)

1.0 g (3.45 mmol) of (+-)-trans-4-(4-dimethylaminophenyl)-3-hydroxymethyl-1-butylpiperidine, 0.51 g (3.45 mmol) phthalimide and 0.9 g (3.45 mmol) of triphenylphosphine were dissolved in 50 ml dry tetrahydrofuran (THF), and 0.60 g (3.45 mmol) of diethylazodicarboxylate (DEAD) was slowly added. After 8 days the solution was evaporated, the solid treated with 2 N NaOH and ether, and the dried (MgSO₄) ether phase was evaporated. The solid was then purified twice by column chromatography on silica gel, first in dichloromethane:methanol (9:1), then in pentane:triethylamine (1:1). The product was then dissolved in acetone and precipitated by the addition of conc. HCl to give 1.0 g of (21), (59%).

C H N Cl analysis: (calc./found for (C₂₆H₃₃N₃O₂, 2 HCl, ½ H₂O)), (62.27/62.17, 7.24/7.35, 8.38/8.28, 14.14/14.19).

### EXAMPLE 17

### (+-)-trans-1-Butyl-4-(4-dimethylaminophenyl)-3-succinimidomethyl-piperidine, HCl (22)

1 g (3.45 mmol) of (+-)-trans-4-(4-dimethylaminophenyl)-3-hydroxymethyl-1-butylpiperidine, 0.34 g (3.45 mmol) succinimide and 0.9 g (3.45 mmol) triphenylphosphine were dissolved in 50 ml dry tetrahydrofuran (THF), and 0.60 g (3.45 mmol) of diethylazodicarboxylate (DEAD) was slowly added. After 5 days the solvent was evaporated, and the solid purified by column chromatography on silica gel in dichloromethane:methanol (19:1). The product was dissolved in acetone and precipitated by the addition of conc. HCl and ether to give 830 mg of (22), 54%.

C H N Cl analysis: (calc./found for (C₂₂H₃₃N₃O₂, 2 HCl, 1½ H₂O)), (56.05/56.30, 8.12/7.98, 8.91/8.91, 15.04/14.70).

## Claims

1. A compound of formula I 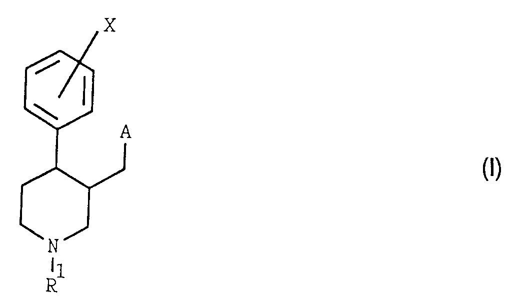 wherein
A is 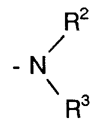 or - O - R⁴
wherein R² and R³ independently are hydrogen, straight or branched C₁₋₆-alkyl optionally substituted with an amino group which amino group is optionally mono or disubstituted with straight or branched C₁₋₆-alkyl, straight or branched C₂₋₆-alkenyl or straight or branched C₂₋₆-alkynyl; or
R² and R³ together form a 5 or 6 membered heterocyclic ring containing one to four N, O or S atom(s) or a combination thereof which heterocyclic ring is optionally substituted at a carbon or nitrogen atom with straight or branched C₁₋₆-alkyl, straight or branched C₂₋₆-alkenyl, straight or branched C₂₋₆-alkynyl or phenyl which phenyl group is optionally substituted with halogen, C₁₋₄-alkyl, C₁₋₄-alkoxy or trifluoromethyl, or one or two carbon atom(s) in the heterocyclic ring together with one or two oxygen atom(s) form one or two carbonyl group(s), or which heterocyclic ring is optionally fused with a benzo group; and
R⁴ is straight or branched C₁₋₆-alkyl optionally substituted with an amino group which amino group is optionally mono or disubstituted with straight or branched C₁₋₆-alkyl, straight or branched C₂₋₆-alkenyl or C₂₋₆-alkynyl; and R¹ is hydrogen, straight or branched C₁₋₆-alkyl optionally substituted with halogen, C₁₋₆-alkoxy, phenyl or phenoxy, straight or branched C₂₋₆-alkenyl or straight or branched C₂₋₆-alkynyl; and
X is hydrogen, halogen, cyano, C₁₋₄-alkyl, C₁₋₄-alkoxy, hydroxy, nitro, trifluoromethyl or an amino group optionally mono or disubstituted with straight or branched C₁₋₆-alkyl, or a pharmaceutically acceptable salt thereof.

2. The compound according to claim 1 selected from
(+-)-trans-3-((2-Aminoethyl)aminomethyl)-1-methyl-4-phenylpiperidine,
(+-)-trans-3(N-Butyl-N-(2-dibutylamino)ethyl)aminomethyl-1-methyl-4-phenylpiperidine,
(+-)-trans-3-((2-Aminoethyl)aminomethyl)-4-(4-fluorophenyl)-1-pentylpiperidine,
(+-)-trans-3-(N-Butyl-N-(2-dibutylamino)ethyl)-aminomethyl-4-(4-fluorophenyl)-1-pentylpiperidine,
(+-)-trans-4-(4-Fluorophenyl)-3-(N-propyl-N-(2-dipropylamino)ethyl)-aminomethyl-1-pentylpiperidine,
(-)-trans-3-((3-Aminopropyl)aminomethyl)-4-(4-fluorophenyl)-1-pentylpiperidine,
(-)-trans-1-Butyl-4-(4-fluorophenyl)-3-(morpholinomethyl)piperidine,
(+-)-trans-1-Butyl-3-(4-phenylpiperidinomethyl)-4-phenylpiperidine,
(+-)-trans-1-Methyl-4-phenyl-3-(1-piperazinyl)methyl-piperidine,
(+-)-trans-4-(4-Fluorophenyl)-3-(4-phenyl-1-piperazinylmethyl)-1-pentylpiperidine,
(-)-trans-4-(4-Fluorophenyl)-3-(1,2,3,4-tetrahydro-2-isoquinolinylmethyl)-1-pentylpiperidine,
(-)-trans-4-(4-Fluorophenyl)-3-(1,2,3,4-tetrahydro-1-quinolinylmethyl)-1-pentylpiperidine,
(-)-trans-3-(2-(Dimethylamino)ethoxymethyl)-4-(4-fluorophenyl)-1-pentylpiperidine,
(+-)-trans-4-(4-Fluorophenyl)-1-methyl-3-phthalimidomethyl-piperidine,
(+-)-trans-4-(4-Fluorophenyl)-1-methyl-3-succinimidomethyl-piperidine,
(+-)-trans-1-Butyl-4-(4-dimethylaminophenyl)-3-phthalimidomethyl-piperidine,
(+-)-trans-1-Butyl-4-(4-dimethylaminophenyl)-3-succinimidomethyl-piperidine,
or a pharmaceutically acceptable salt thereof.

3. A method of preparing a compound according to claim 1 characterized in
a) reacting a compound of formula II 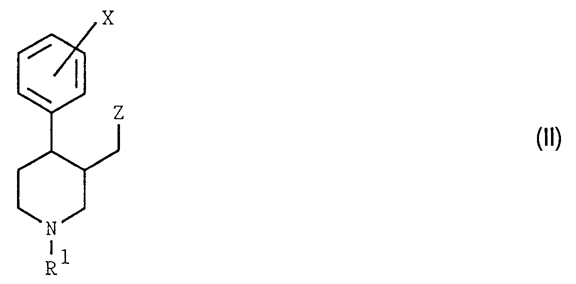 wherein X and R¹ have the meanings defined above provided that R¹ is not hydrogen and Z is a leaving group such as e.g. halogen or a sulfonate, with a compound of formula III 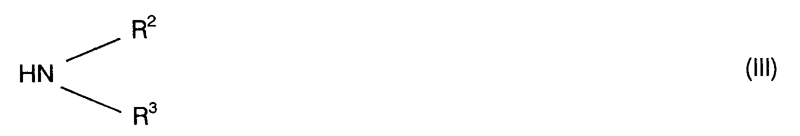 wherein R² and R³ have the meanings defined above; or
b) reacting a compound of formula II 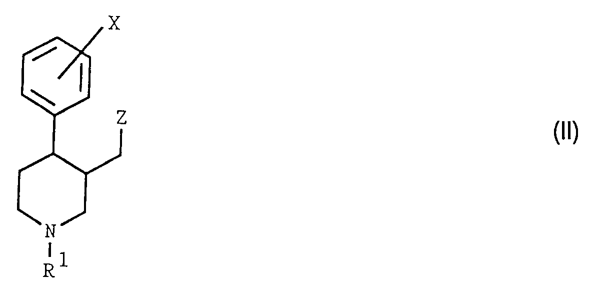 wherein X and R¹ have the meanings defined above provided that R¹ is not hydrogen and Z is a leaving group such as e.g. halogen or a sulfonate, with a compound of formula IV
R⁴ - OH (IV)
wherein R⁴ has the meaning defined above; or
c) demethylating a compound of formula I wherein R¹ is methyl by means of ROCOCl wherein R preferably are -CHClCH₃, -CH=CH₂ and -CH₂CCl₃ to form a compound of formula I wherein R¹ is hydrogen; or
d) reacting a compound of formula V 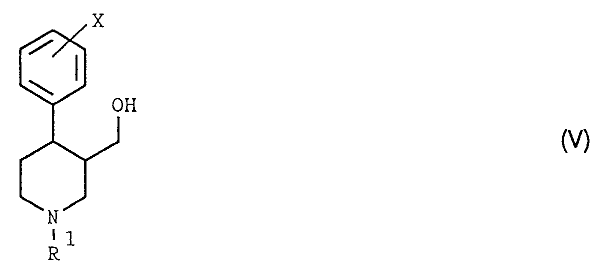 wherein X and R¹ have the meanings defined above with a compound of formula III 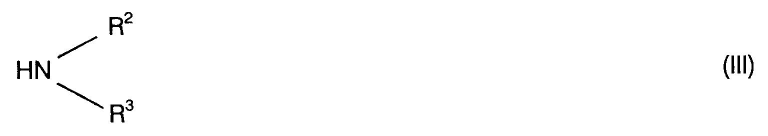 wherein R² and R³ have the meanings defined above by means of Ph₃P and (EtOCON)₂; or
e) reacting a compound of formula V 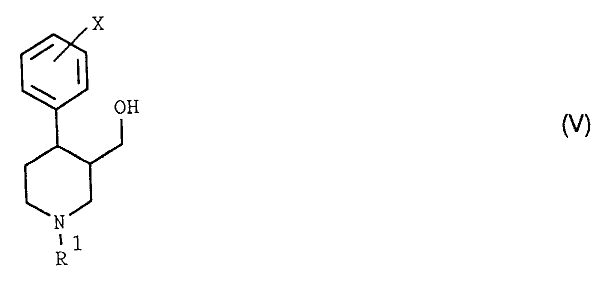 wherein X and R¹ have the meanings defined above with a compound of formula IV
R⁴ - OH (IV)
wherein R⁴ has the meaning defined above by means of Ph₃P and (EtOCON)₂; or
f) reacting a compound of formula VI 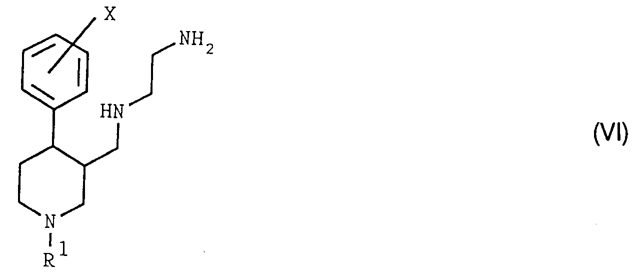 wherein X and R¹ have the meanings defined above provided that R¹ is not hydrogen with a compound of formula VII
R² - Y (VII)
wherein R² is straight or branched C₁₋₆-alkyl and Y is a leaving group such as e.g. halogen or a sulfonate; or
g) reacting a compound of formula VI 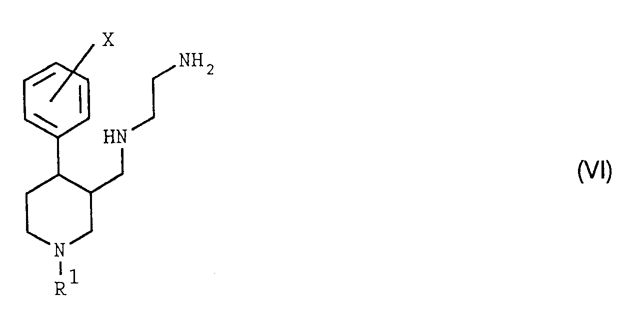 wherein X and R¹ have the meanings defined above provided that R¹ is not hydrogen with a compound of formula VIII
Y-CH₂CH₂-Y (VIII)
wherein Y is a leaving group such as e.g. halogen or a sulfonate.

4. A pharmaceutical composition comprising a compound of claim 1 or a salt thereof with a pharmaceutically acceptable acid together with a pharmaceutically acceptable carrier or diluent.

5. A pharmaceutical composition suitable for use in preventing calcium overload in brain cells of mammals, including humans, comprising an amount of a compound of claim 1, which is effective for inhibiting calcium uptake into brain cells together with a pharmaceutically acceptable carrier or diluent.

6. The pharmaceutical composition according to claim 4 or 5 wherein it is in the form of an oral dosage unit containing 1-100 mg of the active compound.

7. A method of treating calcium overload in brain cells of mammals, including humans, comprising administering a calcium overload blocking amount of a compound according to claim 1.

8. A method of treating calcium overload in brain cells of mammals, including humans, comprising administering a pharmaceutical composition according to claim 5.

9. The use of a compound according to claim 1 or a salt thereof with a pharmaceutically acceptable acid for the preparation of a medicament useful in treatment of calcium overload in brain cells of mammals, including humans.
